(19) Europäisches Patentamt
European Patent Office
Office européen des brevets

(11) EP 2 258 361 B1

(12) **EUROPEAN PATENT SPECIFICATION**

(45) Date of publication and mention of the grant of the patent:
**17.09.2014 Bulletin 2014/38**

(21) Application number: **09724165.7**

(22) Date of filing: **25.03.2009**

(51) Int Cl.:
*A61K 31/12* (2006.01) *A61K 31/194* (2006.01)
*A61K 31/423* (2006.01) *A61K 31/5377* (2006.01)
*A61K 39/395* (2006.01) *A61K 45/00* (2006.01)
*A61K 45/06* (2006.01) *A61P 1/00* (2006.01)
*A61P 1/16* (2006.01) *A61P 13/12* (2006.01)
*A61P 19/02* (2006.01) *A61P 29/00* (2006.01)
*A61P 37/02* (2006.01) *A61P 37/06* (2006.01)
*A61P 43/00* (2006.01) *C07D 261/20* (2006.01)

(86) International application number:
**PCT/JP2009/055930**

(87) International publication number:
**WO 2009/119652 (01.10.2009 Gazette 2009/40)**

(54) **USE OF BENZOPHENONE DERIVATIVE OR SALT THEREOF AND TNFalpha INHIBITOR IN COMBINATION AND PHARMACEUTICAL COMPOSITION CONTAINING THE DERIVATIVE OR SALT THEREOF AND THE INHIBITOR**

VERWENDUNG EINES BENZOPHENONDERIVATS ODER EINES SALZES DARAUS IN KOMBINATION MIT EINEM TNFalpha-HEMMER SOWIE PHARMAZEUTISCHE ZUSAMMENSETZUNG AUS DEM DERIVAT ODER EINEM SALZ DARAUS UND DEM HEMMER

UTILISATION D'UN DÉRIVÉ DE BENZOPHÉNONE OU D'UN SEL DE CELUI-CI ET INHIBITEUR DE TNFalpha EN COMBINAISON ET COMPOSITION PHARMACEUTIQUE CONTENANT LE DÉRIVÉ OU LE SEL DE CELUI-CI ET L'INHIBITEUR

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **27.03.2008 JP 2008083730**

(43) Date of publication of application:
**08.12.2010 Bulletin 2010/49**

(73) Proprietor: **Toyama Chemical Co., Ltd.**
**Shinjuku-ku**
**Tokyo 160-0023 (JP)**

(72) Inventors:
- **AIKAWA, Yukihiko**
**Toyama-shi**
**Toyama 930-8508 (JP)**
- **SHIOZAWA, Shunichi**
**Kobe-shi**
**Hyogo 651-2274 (JP)**

(74) Representative: **Blodig, Wolfgang et al**
**Wächtershäuser & Hartz**
**Patentanwaltspartnerschaft mbB**
**Weinstrasse 8**
**80333 München (DE)**

(56) References cited:
**EP-A1- 1 445 249** **WO-A1-03/042150**
**WO-A2-2004/037205** **WO-A2-2004/060911**
**JP-T- 2006 507 286** **JP-T- 2006 517 191**

- **O'DELL, J.R. ET AL.: 'Treatment of rheumatoid arthritis with methotrexate alone, sulfasalazine and hydroxychloroquine, or a combination of all three medications' N.ENGL.J.MED. vol. 334, no. 20, 1996, pages 1287 - 1291, XP008141323**
- **AIKAWA, Y. ET AL.: 'Treatment of arthritis with a selective inhibitor of c-Fos/activator protein-1' NAT BIOTECHNOL vol. 26, no. 7, July 2008, pages 817 - 823, XP008141319**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a method of using a benzophenone derivative or a salt thereof and a TNFα inhibitor in combination for the treatment such as the cure or prevention of autoimmune diseases. In addition, the present invention also relates to a pharmaceutical composition containing a benzophenone derivative or a salt thereof and a TNFα inhibitor useful for the treatment such as the cure or prevention of autoimmune diseases.

### BACKGROUND ART

Autoimmune diseases, such as arthritis diseases in connective tissue disorders typified by rheumatoid arthritis cause, for example, dysfunction as a result of the progression of the destruction of cartilage and/or bone, and thus, this disease largely affects daily life.

To date, for the drug treatment of rheumatoid arthritis and other types of arthritis, nonsteroidal anti-inflammatory drugs (NSAIDs) such as aspirin and indomethacin, gold preparation, disease-modifying anti-rheumatoid drugs (DMARDs) such as D-penicillamine, immunosuppressive agents such as methotrexate, steroidal drugs, and the like have been used. However, the progression of the destruction of cartilage and/or bone, which is the largest problem of arthritis, cannot be completely suppressed with the currently used treatment methods. Moreover, from the viewpoint of side effects, the aforementioned drugs cannot be used for a long period of time. Thus, these treatment methods have not yet provided a satisfactory treatment.

As a drug exhibiting effects on autoimmune diseases, TNFα inhibitors have been known, for example (Non-Patent Document 1). The TNFα inhibitors suppress arthritis through inhibiting the action of TNFα that is an important cytokine associated with synovitis and the destruction of cartilage and/or bone. As such TNFα inhibitors, an anti-TNFα antibodies and a soluble TNFα receptor have been placed on the market, and even at present, the research and development of drugs are being performed.

On the other hand, benzophenone derivatives having an antiarthritic action have been known. It has been known that these benzophenone derivatives inhibit a transcription factor AP-1, and as a result, have an excellent antiarthritic action (Patent Document 1).

Moreover, a method of using several anti-arthritis agents in combination has been known (Non-Patent Document 2). However, the number of anti-arthritis agents for such combined use is limited, and thus, satisfactory therapeutic effects have not been achieved.

Furthermore, a method of using a TNFα inhibitor and a benzophenone derivative having an antiarthritic action in combination has not been known at all.

PATENT DOCUMENT 1: International Publication No. WO03/042150 pamphlet
NON-PATENT DOCUMENT 1: The New England Journal of Medicine (N. Engl. J. Med.), vol. 355, pp. 704-712 (2006)
NON-PATENT DOCUMENT 2: The New England Journal of Medicine (N. Engl. J. Med.), vol. 334, pp. 1287-1291 (1996)

### DISCLOSURE OF THE INVENTION

### PROBLEMS TO BE SOLVED BY THE INVENTION

It has been desired to develop a method useful for the treatment such as the cure or prevention of autoimmune diseases, and a pharmaceutical composition useful for the treatment such as the cure or prevention of autoimmune diseases.

### MEANS FOR SOLVING THE PROBLEMS

Under the aforementioned circumstances, as a result of intensive studies, the present inventors have discovered that a method of using a benzophenone derivative represented by the general formula [1] below or a salt thereof and one or more TNFα inhibitors in combination is useful as a method for the treatment such as the cure or prevention of autoimmune diseases:

[Formula 1]

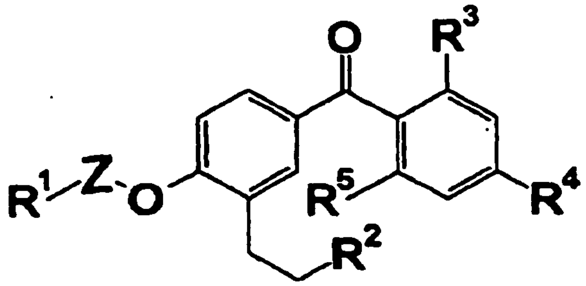

[1]

wherein $R^1$ represents a heterocyclic group which may be substituted, a substituted phenyl group or an alkyl group which may be substituted; Z represents an alkylene group which may be substituted; $R^2$ represents a heterocyclic group which may be substituted, an alkoxycarbonyl or heterocyclic carbonyl group which may be substituted, or a carboxyl group which may be protected; $R^3$ represents a hydrogen atom, a halogen atom, a cyano group, a nitro group, a carboxyl group which may be protected, a hydroxyl group which may be protected, an amino group which may be protected, a mercapto group, a carbamoyl group, or an alkyl, alkenyl, cycloalkyl, aryl, aralkyl, alkoxy, aryloxy, acyl, alkoxycarbonyl, aryloxycarbonyl, alkylthio, alkylsulfinyl, alkylsulfonyl, alkylamino, acylamino, alkylsulfonylamino, arylsulfonylamino or heterocyclic group which may be substituted; $R^4$ represents a cycloalkyloxy, which may be substituted; and $R^5$ represents a hydrogen atom, a halogen atom, or a hydroxyl group. Further, the inventors have discovered that a pharmaceutical composition containing these substances is useful for the treatment such as the cure or prevention of autoimmune diseases. Thus, the inventors have completed the present invention.

ADVANTAGES OF THE INVENTION

The method of using the benzophenone derivative represented by the general formula [1] or the salt thereof and one or more TNFα inhibitors in combination is useful as a method for the treatment such as the cure or prevention of autoimmune diseases, and the pharmaceutical composition containing these substances is useful for the treatment such as the cure or prevention of autoimmune diseases.

BEST MODE FOR CARRYING OUT THE INVENTION

The present invention will be described in detail below.

In the present description, each term has the following meanings, unless otherwise specified.

A halogen atom refers to a fluorine atom, a chlorine atom, a bromine atom and an iodine atom; an alkyl group refers to, for example, a linear or branched $C_{1-12}$ alkyl group such as methyl, ethyl, n-propyl, isopropyl, n-butyl, sec-butyl, isobutyl, tert-butyl, pentyl, isopentyl, hexyl, heptyl and octyl; a lower alkyl group refers to, for example, a linear or branched $C_{1-6}$ alkyl group such as methyl, ethyl, n-propyl, isopropyl, n-butyl, sec-butyl, isobutyl, tert-butyl, pentyl and isopentyl; a halogeno lower alkyl group refers to, for example, a linear or branched halogeno-$C_{1-6}$ alkyl group such as fluoromethyl, chloromethyl, bromomethyl, dichloromethyl, trifluoromethyl, trichloromethyl, chloroethyl, dichloroethyl, trichloroethyl and chloropropyl; a lower alkoxy lower alkyl group refers to, for example, a linear or branched $C_{1-6}$ alkoxy-$C_{1-6}$ alkyl group such as methoxymethyl, ethoxymethyl, n-propoxymethyl, methoxyethyl and ethoxyethyl; a hydroxy lower alkyl group refers to, for example, a linear or branched hydroxy-$C_{1-6}$ alkyl group such as hydroxymethyl, hydroxyethyl and hydroxypropyl; and an amino lower alkyl group refers to, for example, an amino-$C_{1-6}$ alkyl group such as aminomethyl, aminoethyl and aminopropyl.

An alkenyl group refers to, for example, a linear or branched $C_{2-12}$ alkenyl group such as vinyl, allyl, propenyl, isopropenyl, butenyl, isobutenyl, pentenyl, hexenyl, heptenyl and octenyl; and a lower alkenyl group refers to, for example, a linear or branched $C_{2-6}$ alkenyl group such as vinyl, allyl, propenyl, isopropenyl, butenyl, isobutenyl and pentenyl.

A cycloalkyl group refers to, for example, a $C_{3-7}$ cycloalkyl group such as cyclopropyl, cyclobutyl, cyclopentyl, cyclohexyl and cycloheptyl; a cycloalkyloxy group refers to, for example, a $C_{3-7}$ cycloalkyloxy group such as cyclopropyloxy, cyclobutyloxy, cyclopentyloxy, cyclohexyloxy and cyclopentyloxy; and a cycloalkenyloxy group refers to, for example, a $C_{5-7}$ cycloalkenyloxy group such as cyclopentenyloxy and cyclohexenyloxy.

An aryl group refers to, for example, phenyl, tolyl and naphthyl; and an aralkyl group refers to, for example, an ar-$C_{1-12}$ alkyl group such as benzyl, diphenylmethyl, trityl, phenethyl, 4-methylbenzyl and naphthylmethyl.

An aryloxy group refers to, for example, phenoxy and naphthoxy; and an aryloxycarbonyl group refers to, for

example, phenoxycarbonyl and naphthoxycarbonyl.

An alkoxy group refers to, for example, a linear or branched $C_{1-12}$ alkoxy group such as methoxy, ethoxy, n-propoxy, isopropoxy, n-butoxy, isobutoxy, sec-butoxy, tert-butoxy, pentyloxy, isopentyloxy, hexyloxy, heptyloxy and octyloxy; a lower alkoxy group refers to, for example, a linear or branched $C_{1-6}$ alkoxy group such as methoxy, ethoxy, n-propoxy, isopropoxy, n-butoxy, isobutoxy, sec-butoxy, tert-butoxy, pentyloxy and isopentyloxy; and an alkoxyalkyl group refers to, for example, methoxymethyl, ethoxymethyl and 2-(trimethylsilyl)ethoxymethyl.

An alkylene group refers to, for example, a linear or branched $C_{1-12}$ alkylene group such as methylene, ethylene and propylene.

An alkoxycarbonyl group refers to, for example, a linear or branched $C_{1-12}$ alkoxycarbonyl group such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl and pentyloxycarbonyl; a lower alkoxycarbonyl group refers to, for example, a linear or branched $C_{1-6}$ alkyloxycarbonyl group such as methoxycarbonyl, ethoxycarbonyl and propoxycarbonyl; a lower alkoxycarbonyl lower alkyl group refers to, for example, a linear or branched $C_{1-6}$ alkoxycarbonyl-$C_{1-6}$ alkyl group such as methoxycarbonylmethyl, ethoxycarbonylmethyl, n-propoxycarbonylmethyl, methoxycarbonylethyl and ethoxycarbonylethyl; and an aralkyloxycarbonyl group refers to, for example, an ar-$C_{1-12}$ alkyloxycarbonyl group such as benzyloxycarbonyl and 4-methylbenzyloxycarbonyl.

A lower alkoxyimino group refers to, for example, a linear or branched $C_{1-6}$ alkoxyimino group such as methoxyimino and ethoxyimino; an alkylamino group refers to, for example, a linear or branched $C_{1-12}$ alkylamino group such as methylamino, ethylamino, propylamino, butylamino, pentylamino, hexylamino, heptylamino and octylamino; a lower alkylamino group refers to, for example, a linear or branched mono- or di-$C_{1-6}$ alkylamino group such as methylamino, ethylamino, propylamino, dimethylamino, diethylamino and methylethylamino; a lower alkylamino lower alkyl group refers to, for example, a mono- or di-$C_{1-6}$ alkylamino-$C_{1-6}$ alkyl group such as methylaminomethyl, methylaminoethyl, ethylaminomethyl, methylaminopropyl, propylaminoethyl, dimethylaminomethyl, diethylaminomethyl, diethylaminoethyl and dimethylaminopropyl; and a lower alkylidene group refers to, for example, a $C_{1-6}$ alkylidene group such as methylene, ethylidene, propylidene and isopropylidene.

A nitrogen-containing heterocyclic group refers to, for example, a 5- or 6-membered ring, condensed ring, or crosslinked ring heterocyclic group, which contains one or more nitrogen atoms as heteroatoms for forming the ring and which may further contain one or more oxygen atoms or sulfur atoms, such as pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, quinazolyl, thiazolyl, tetrazolyl, thiadiazolyl, pyrrolinyl, imidazolinyl, imidazolidinyl, pyrazolinyl, pyrazolidinyl, purinyl and indazolyl groups.

A heterocyclic group refers to, the aforementioned nitrogen-containing heterocyclic groups, and also, for example, a 5- or 6-membered ring, condensed ring, or crosslinked ring heterocyclic group, which contains at least one heteroatom selected from nitrogen, oxygen and sulfur atoms, and which may contain one or more oxygen atoms or sulfur atoms as heteroatoms for forming the ring, such as furyl, thienyl, 4-methyl-2-oxo-1,3-dioxole, benzothienyl, pyranyl, isobenzofuranyl, oxazolyl, benzofuranyl, indolyl, benzimidazolyl, benzoxazolyl, benzisoxazolyl, benzothiazolyl, quinoxalyl, dihydroquinoxalinyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, thiadiazolyl, oxadiazolyl, pyridazinyl, isoindolyl and isoquinolyl groups; and a heterocyclic carbonyl group refers to, for example, a heterocyclic -CO- group such as 4-hydroxy-2-(5H)-furanocarbonyl, morpholinocarbonyl, piperazinocarbonyl and pyrrolidinocarbonyl groups.

An acyl group refers to, for example, a formyl group, a linear or branched $C_{2-12}$ alkanoyl group such as acetyl, isovaleryl, propionyl and pivaloyl, an aralkylcarbonyl group such as benzylcarbonyl, an aroyl group such as benzoyl and naphthoyl, and a heterocyclic carbonyl group such as nicotinoyl, thenoyl, pyrrolidinocarbonyl and furoyl groups; and an acylamino group refers to, for example, a $C_{1-6}$ acylamino group such as formylamino, acetylamino, propionylamino and butyrylamino.

A cyclic amino group may refer to, for example, any of saturated cyclic amino and unsaturated cyclic amino groups, and it may further contain one or more heteroatoms such as nitrogen atoms, oxygen atoms and sulfur atoms and carbonyl carbons in the ring thereof, and it may also be a monocyclic, bicyclic or tricyclic group. More specifically, such cyclic amino group refers to: a saturated or unsaturated monocyclic 3- to 7-membered cyclic amino group having one nitrogen atom, such as aziridin-1-yl, azetidin-1-yl, pyrrolidin-1-yl, pyrrolin-1-yl, pyrrol-1-yl, dihydropyridin-1-yl, piperidin-1-yl, dihydroazepin-1-yl and perhydroazepin-1-yl; a saturated or unsaturated monocyclic 3- to 7-membered cyclic amino group having two nitrogen atoms, such as imidazol-1-yl, imidazolidin-1-yl, imidazolin-1-yl, pyrazolidin-1-yl, piperazin-1-yl, 1,4-dihydropyrazin-1-yl, 1,2-dihydropyrimidin-1-yl, perhydropyrazin-1-yl and homopiperazin-1-yl; a saturated or unsaturated monocyclic 3- to 7-membered cyclic amino group having three or more nitrogen atoms, such as 1,2,4-triazol-1-yl, 1,2,3-triazol-1-yl, 1,2-dihydro-1,2,4-triazin-1-yl and perhydro-S-triazin-1-yl; a saturated or unsaturated monocyclic 3-to 7-membered cyclic amino group having 1 to 4 heteroatoms selected from oxygen atoms and sulfur atoms, in addition to nitrogen atoms, such as oxazolidin-3-yl, isoxazolidin-2-yl, morpholin-4-yl, thiazolidin-3-yl, isothiazolidin-2-yl, thiomorpholin-4-yl, homothiomorpholin-4-yl and 1,2,4-thiadiazolin-2-yl; a saturated or unsaturated, bicyclic or tricyclic amino group, such as isoindolin-2-yl, indolin-1-yl, 1H-indazol-1-yl, purin-7-yl and tetrahydroquinolin-1-yl; and a spiro or crosslinked, saturated or unsaturated 5- to 12-membered cyclic amino group, such as 5-azaspiro[2.4]heptan-5-yl, 2,8-diazabicyclo[4.3.0]nonan-8-yl, 3-azabicyclo[3.1.0]hexan-3-yl, 2-oxa-5,8-diazabicyclo[4.3.0]nonan-8-yl, 2,8-diazaspiro[4.4]nonan-2-yl and 7-azabicyclo[2.2.1]heptan-7-yl.

An alkylthio group refers to, for example, a linear or branched $C_{1-12}$ alkylthio group such as methylthio, ethylthio, n-propylthio, isopropylthio, n-butylthio, isobutylthio, sec-butylthio, tert-butylthio, pentylthio, isopentylthio, hexylthio, heptylthio and octylthio; and a lower alkylthio group refers to, for example, a linear or branched $C_{1-6}$ alkylthio group such as methylthio, ethylthio, n-propylthio, isopropylthio, n-butylthio, isobutylthio, sec-butylthio, tert-butylthio, pentylthio and isopentylthio.

An alkylsulfinyl group refers to, for example, a linear or branched $C_{1-12}$ alkylsulfinyl group such as methylsulfinyl, ethylsulfinyl, n-propylsulfinyl, isopropylsulfinyl, n-butylsulfinyl, isobutylsulfinyl, sec-butylsulfinyl, tert-butylsulfinyl, pentylsulfinyl, isopentylsulfinyl, hexylsulfinyl, heptylsulfinyl and octylsulfinyl; an alkylsulfonyl group refers to, for example, a linear or branched $C_{1-12}$ alkylsulfonyl group such as methylsulfonyl, ethylsulfonyl, n-propylsulfonyl, isopropylsulfonyl, n-butylsulfonyl, isobutylsulfonyl, sec-butylsulfonyl, tert-butylsulfonyl, pentylsulfonyl, isopentylsulfonyl, hexylsulfonyl, heptylsulfonyl and octylsulfonyl; and an arylsulfonyl group refers to, for example, benzenesulfonyl and p-toluenesulfonyl.

An alkylsulfonylamino group refers to, for example, a linear or branched $C_{1-12}$ alkylsulfonylamino group such as methylsulfonylamino, ethylsulfonylamino, n-propylsulfonylamino, isopropylsulfonylamino, n-butylsulfonylamino, isobutylsulfonylamino, sec-butylsulfonylamino, tert-butylsulfonylamino, pentylsulfonylamino, isopentylsulfonylamino, hexylsulfonylamino, heptylsulfonylamino and octylsulfonylamino; and an arylsulfonylamino group refers to, for example, an aryl-$SO_2$NH- group such as phenylsulfonylamino and naphthylsulfonylamino.

A lower alkylsulfinyl group refers to, for example, a linear or branched $C_{1-6}$ alkylsulfinyl group such as methylsulfinyl, ethylsulfinyl, n-propylsulfinyl, isopropylsulfinyl, n-butylsulfinyl, isobutylsulfinyl, sec-butylsulfinyl, tert-butylsulfinyl, pentylsulfinyl and hexylsulfinyl; and a lower alkylsulfonyl group refers to, for example, a linear or branched $C_{1-6}$ alkylsulfonyl group such as methylsulfonyl, ethylsulfonyl, n-propylsulfonyl, isopropylsulfonyl, n-butylsulfonyl, isobutylsulfonyl, sec-butylsulfonyl, tert-butylsulfonyl and pentylsulfonyl.

A lower alkylcarbamoyl group refers to, for example, a mono- or di-$C_{1-6}$ alkylcarbamoyl group such as methylcarbamoyl, ethylcarbamoyl, propylcarbamoyl, dimethylcarbamoyl, diethylcarbamoyl and methylethylcarbamoyl; and a lower alkylsulfonylamino group refers to, for example, a linear or branched $C_{1-6}$ alkylsulfonylamino group such as methylsulfonylamino, ethylsulfonylamino, n-propylsulfonylamino, isopropylsulfonylamino, n-butylsulfonylamino, isobutylsulfonylamino, sec-butylsulfonylamino, tert-butylsulfonylamino and pentylsulfonylamino.

A lower alkylsulfonylcarbamoyl group refers to, for example, a linear or branched $C_{1-6}$ alkylsulfonylcarbamoyl group such as methylsulfonylcarbamoyl, ethylsulfonylcarbamoyl, n-propylsulfonylcarbamoyl, isopropylsulfonylcarbamoyl, n-butylsulfonylcarbamoyl, isobutylsulfonylcarbamoyl, sec-butylsulfonylcarbamoyl, tert-butylsulfonylcarbamoyl and pentylsulfonylcarbamoyl; and a lower alkylaminosulfonyl group refers to, for example, a mono-or di-$C_{1-6}$ alkylaminosulfonyl group such as methylaminosulfonyl, ethylaminosulfonyl, propylaminosulfonyl, dimethylaminosulfonyl, diethylaminosulfonyl and methylethylaminosulfonyl.

A carboxyl lower alkenyl group refers to, for example, a linear or branched $C_{2-6}$ alkenyl group substituted with a carboxyl group.

A lower alkyl heterocyclic group refers to, for example, a heterocyclic group substituted with a linear or branched lower alkyl group; and a hydroxy heterocyclic group refers to, for example, a heterocyclic group substituted with a hydroxyl group.

A lower alkoxy lower alkoxy group refers to a linear or branched $C_{1-6}$ alkoxy group substituted with a lower alkoxy group.

A heterocyclic-oxy group refers to groups represented by heterocyclic -O-, bound via oxygen atoms, such as pyrrolidinyloxy, piperidinyloxy, tetrahydrofuranyloxy, tetrahydropyranyloxy and tetrahydrothiopyranyloxy.

A carboxyl protective group includes any group which can be normally used as a protective group of a carboxyl group, for example, the groups described in W. Greene et al. "Protective Groups in Organic Synthesis" Third Edition, pp. 369 to 453, 1999, John Wiley & Sons, INC. More specifically, examples of a carboxyl protective group include an alkyl group, an alkenyl group, an aryl group, an aralkyl group, a cycloalkyl group and an alkoxyalkyl group.

An amino protective group includes any group which can be normally used as a protective group of an amino group, for example, the groups described in W. Greene et al. "Protective Groups in Organic Synthesis" Third Edition, pp. 494 to 615, 1999, John Wiley & Sons, INC. More specifically, examples of an amino protective group include an acyl group, an alkoxycarbonyl group, an aralkyloxycarbonyl group, an aryloxycarbonyl group, an aralkyl group, an alkoxyalkyl group, an alkylsulfonyl group and an arylsulfonyl group.

A hydroxyl protective group includes any group which can be normally used as a protective group of a hydroxyl group, for example, the groups described in W. Greene et al. "Protective Groups in Organic Synthesis" Third Edition, pp. 17 to 245, 1999, John Wiley & Sons, INC. More specifically, examples of a hydroxyl protective group include an acyl

group, an alkoxycarbonyl group, an aralkyloxycarbonyl group, an alkyl group, an alkenyl group, an aralkyl group and an alkoxyalkyl group.

Each of the heterocyclic, phenyl and alkyl groups represented by $R^1$; the heterocyclic, alkoxycarbonyl and heterocyclic carbonyl groups represented by $R^2$; the alkyl, alkenyl, cycloalkyl, aryl, aralkyl, alkoxy, aryloxy, acyl, alkoxycarbonyl, aryloxycarbonyl, alkylthio, alkylsulfinyl, alkylsulfonyl, alkylamino, acylamino, alkylsulfonylamino, arylsulfonylamino and heterocyclic groups represented by $R^3$; and the alkoxy, cycloalkyloxy, cycloalkenyloxy, alkyl, cycloalkyl, heterocyclic-oxy and heterocyclic groups represented by $R^4$ may be further substituted with one or more groups selected from a cyano group, a nitro group, a halogen atom, carboxyl, phosphoryl, hydroxyl, amino, carbamoyl, hydroxycarbamoyl, aminosulfonyl, sulfo, hydroxy lower alkyl, amino lower alkyl, cyclic amino, lower alkylamino and lower alkylamino lower alkyl groups which may be protected, a lower alkyl group, a lower alkenyl group, a lower alkoxy group, a lower alkoxycarbonyl group, an acyl group, an aryl group, a heterocyclic group, a cycloalkyl group, an aralkyl group, a lower alkylidene group, a mercapto group, a lower alkylthio group, a lower alkylsulfinyl group, a lower alkylsulfonyl group, a lower alkylsulfonylcarbamoyl group, a lower alkylcarbamoyl group, a lower alkylsulfonylamino group, a lower alkylaminosulfonyl group, a carboxyl lower alkenyl group, a hydroxy heterocyclic group, a lower alkyl heterocyclic group, a lower alkoxy lower alkoxy group, a halogeno lower alkyl group, a lower alkoxy lower alkyl group, a lower alkoxycarbonyl lower alkyl group, and a lower alkoxyimino group.

The alkylene group represented by Z may be further substituted with one or more groups selected from a cyano group, a nitro group, a halogen atom, carboxyl, carbamoyl, hydroxycarbamoyl, hydroxy lower alkyl, amino lower alkyl and lower alkylamino lower alkyl groups which may be protected, a lower alkyl group, a lower alkoxycarbonyl group, an acyl group, an aryl group, a heterocyclic group, a cycloalkyl group, a lower alkenyl group, an aralkyl group, a lower alkylsulfonylcarbamoyl group, a lower alkylcarbamoyl group, a halogeno lower alkyl group, a lower alkoxy lower alkyl group and a lower alkoxycarbonyl lower alkyl group.

The aforementioned each substituent may be further substituted with the groups exemplified as substituents for each substituent.

In addition, the heterocyclic group and cyclic amino group for each substituent may be further substituted with a keto group.

The salt of the compound of the general formula [1] includes commonly known salts formed with a basic group such as an amino group, or with an acidic group such as a hydroxyl or carboxyl group.

Examples of salts formed with a basic group include salts with mineral acid such as hydrochloric acid, hydrobromic acid, nitric acid and sulfuric acid; salts with organic carboxylic acid such as formic acid, acetic acid, citric acid, oxalic acid, fumaric acid, maleic acid, succinic acid, malic acid, tartaric acid, aspartic acid, trichloroacetic acid and trifluoroacetic acid; and salts with sulfonic acid such as methanesulfonic acid, benzenesulfonic acid, p-toluenesulfonic acid, mesitylenesulfonic acid and naphthalenesulfonic acid.

Examples of salts formed with an acidic group include salts with alkaline metal such as sodium and potassium; salts with alkaline earth metal such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine and N,N'-dibenzylethylenediamine.

Moreover, among the above described salts, a preferable salt of the compound of the general formula [1] is a pharmaceutically acceptable salt thereof.

When isomers (for example, optical isomers, geometric isomers, and tautomers) exist in the benzophenone derivative represented by the general formula [1] or the salt thereof, the present invention includes all such isomers, and also includes hydrates, solvates and all crystals.

Preferred compounds as the benzophenone derivative represented by the general formula [1] or the salt thereof are as follows.

The compound wherein $R^1$ is a heterocyclic group which may be substituted or a substituted phenyl group is preferable. The compound wherein $R^1$ is a heterocyclic group which may be substituted is more preferable.

The compound wherein $R^2$ is a carboxyl group which may be protected with an alkyl group is preferable. The compound wherein $R^2$ is a carboxyl group is more preferable.

The compound wherein $R^3$ is a hydroxyl group which may be protected is preferable. The compound wherein $R^3$ is a hydroxyl group is more preferable.

The compound wherein $R^4$ is a cycloalkyloxy group which may be substituted is preferable. The compound wherein $R^4$ is a cycloalkyloxy group is more preferable.

The compound wherein $R^5$ is a hydrogen atom is preferable.

The compound wherein Z is an alkylene group is preferable, and the compound wherein Z is a methylene group is more preferable.

Preferred benzophenone derivatives represented by the general formula [1] include: 2-(4-morpholinyl)ethyl 3-(5-(4-(cyclopentyloxy)-2-hydroxybenzoyl)-2-((3-hydroxy-1,2-benzisoxazol-6-yl)methoxy)phenyl)propionate; 4-((2-(2-carboxyethyl)-4-(4-(cyclopentyloxy)-2-hydroxybenzoyl)phenoxy)methyl)benzoic acid; 3-(5-(4-(cyclopentyloxy)-2-hydroxybenzoyl)-2-((4-(3-hydroxy-5-isoxazolyl)benzyl)oxy)phenyl)propionic acid; and 3-(5-(4-(cyclopentyloxy)-2-hydroxybenzoyl)-2-((3-hydroxy-1,2-benzisoxazol-6-yl)methoxy)phenyl)propionic acid; or the salts thereof Of these, 3-(5-(4-(cyclopentyloxy)-2-hydroxybenzoyl)-2-((3-hydroxy-1,2-benzisoxazol-6-yl)methoxy)phenyl)propionic acid or the salt thereof are more preferable.

The benzophenone derivative represented by the general formula [1] is produced by combining known methods. For example, it can be produced by the method described in Patent Document 1.

The autoimmune diseases in the present invention include: arthritis diseases such as rheumatoid arthritis, juvenile idiopathic arthritis and psoriatic arthritis; inflammatory bowel diseases such as ulcerative colitis and Crohn's disease; systemic lupus erythematosus; scleroderma; Behcet's disease; rheumatic fever; polymyositis; periarteritis nodosa; Sjogren's syndrome; active chronic hepatitis; and glomerular nephritis. Of these diseases, the arthritis diseases are preferable, and rheumatoid arthritis is more preferable.

Examples of the TNF$\alpha$ inhibitor used in the present invention include compounds having a TNF$\alpha$ inhibitory action, such as anti-TNF$\alpha$ antibodies and soluble TNF$\alpha$ receptors. The anti-TNF$\alpha$ antibodies are more preferable.

The administration route of the pharmaceutical composition of the present invention is not particularly limited. The present pharmaceutical composition can be administered via intravenous, oral, intramuscular, subcutaneous, inhalation, spraying, or other administration routes. Moreover, the benzophenone derivative represented by the general formula [1] or the salt thereof may be administered at the same time with the TNF$\alpha$ inhibitor, or in a specific order.

The method of using the benzophenone derivative represented by the general formula [1] or the salt thereof and one or more TNF$\alpha$ inhibitors in combination according to the present invention is useful as a method for the treatment such as the cure or prevention of autoimmune diseases. In addition, this method is more usefully used for the cure of the aforementioned disease.

Moreover, a pharmaceutical composition containing the benzophenone derivative represented by the general formula [1] or the salt thereof and one or more TNF$\alpha$ inhibitors is useful for the treatment such as the cure or prevention of autoimmune diseases. Furthermore, this pharmaceutical composition is more usefully used for the cure of the aforementioned disease.

According to the method and pharmaceutical composition of the present invention, the treatment such as the cure or prevention of more severe autoimmune diseases become possible. Further, even if the amounts of individual agents used are reduced and then administered, the pharmaceutical composition still exhibits a strong action. Thus, it becomes possible to reduce the side effects of individual agents.

When the pharmaceutical composition of the present invention is used, formulation additives such as excipients, carriers and dilution agents, which are generally used for formulation, may be appropriately mixed with the present pharmaceutical composition. According to an ordinary method, these compositions may be formulated as tablets, capsules, powders, syrups, granules, pills, suspensions, emulsions, liquids, powder formulations, suppositories, eye drops, nose drops, ear drops, adhesive skin patches, ointments, injections and the like, and may be administered either orally or parenterally. Moreover, the administration method, dosage and the number of doses of the preparations may be arbitrarily determined in accordance with the age and weight of the patient, and the severity of the patient's symptoms. The recommended dose range for adult patients is generally 0.01 to 1000 mg/kg/day via oral administration or parenteral administration (for example, injection, intravenous drip and rectal administration) either once or divided over several administrations, or by administering the doses for several days at one time.

## EXAMPLES

The present invention will be described in the following test examples. However, these examples are not intended to limit the scope of the present invention.

3-(5-(4-(cyclopentyloxy)-2-hydroxybenzoyl)-2-((3-hydroxy-1,2-benzisoxazol-6-yl)methoxy)phenyl)propionic acid (hereinafter referred to as compound A) was selected as a tested substance. An anti-TNF$\alpha$ antibody (clone No. TN3-19.12, R&D Systems) was selected as a TNF$\alpha$ inhibitor.

### Test Example 1 (Effects of the combined use of compound A and anti-TNF$\alpha$ antibody on mouse type II collagen-induced arthritis)

Eight-week-old male DBA/1J mice (9 or 10 mice per group; Charles River Laboratories Japan) were used. A 2 mg/mL bovine type II collagen dissolved in a 0.1 mol/L acetic acid solution (Koken Co., Ltd.) was mixed with an equal volume of Freund's complete adjuvant (BD Diagnostic Systems) to prepare an emulsion. The emulsion (0.2 mL) was intradermally injected into the hip of each mouse. Twenty-one days after the primary immunization, the same treatment was carried out (secondary sensitization), so that type II collagen arthritis was induced.

Compound A was dissolved in a 2-fold molar amount of sodium hydroxide solution, and a 3-fold weight of

polyvinylpyrrolidone was then added to the solution, followed by dilution with distilled water. The concentration of compound A in a compound A (30 mg/kg)-dosing solution was adjusted to be 3 mg/mL. The concentration of compound A in compound A (3 mg/kg)-dosing solution for combined administration was adjusted to be 0.3 mg/mL. Each dosing solution was orally administered to the mice.

The anti-TNFα antibody was dissolved in phosphate buffered saline. The concentration of the anti-TNFα antibody in the anti-TNFα antibody (250 μg/mouse)-dosing solution was adjusted to be 1.25 mg/mL. The concentration of the anti-TNFα antibody in the anti-TNF α antibody (50 μg/mouse)-dosing solution for combined administration was adjusted to be 0.25 mg/mL. Each dosing solution was intraperitoneally administered to the mice.

To control group, polyvinylpyrrolidone solution was orally administered, an d hamster IgG (MP Biomedical) dissolved in phosphate buffered saline (hamster IgG concentration: 0.25 mg/mL) was intraperitoneally administered.

After the second immunization, mice that developed arthritis were successively divided into groups. Thereafter, from the day at which the onset of arthritis was observed (day 1), compound A was administered once a day for 10 days, and the anti-TNFα antibody was administered on day 1, day 4 and day 8.

The knuckle portion and the articulations of wrist and tarsus portions of the four paws of each mouse were evaluated using the following 4 scores. In a total of the four paws, the maximum arthritis score was set at 12 points.

0: no changes
1: swelling of one or two toes or a slight swelling of ankle
2: swelling of three or more toes, or moderate swelling of ankle
3: extensive swelling of paws

In addition, the arthritis inhibition rate was obtained by the following formula:

$$\text{Arthritis inhibition rate (\%)} = 100 - (\text{the score of the tested substance-dosing group/the score of the control group}) \times 100$$

The results of the arthritis on the day following the final administration are shown in Table 1.

[Table 1]

| Dosing group | Arthritis inhibition rate (%) |
|---|---|
| Compound A (30 mg/kg) | 23 |
| Anti-TNFα antibody 250 μg/mouse | 32 |
| Compound A 3 mg/kg and anti-TNFα antibody 50 μg /mouse | 50 |

The arthritis inhibition rates of compound A (30 mg/kg)-dosing administration group and the anti-TNFα antibody (250 μg /mouse)-dosing administration group were 23% and 32%, respectively. In contrast, the arthritis inhibition rate of the group to which both compound A (3 mg/kg) and the anti-TNFa antibody (50 μg /mouse) were applied in combination, was 50%. Thus, the combined use of compound A and the anti-TNFa antibody extremely strongly inhibited arthritis.

The doses of compound A and the anti-TNFα antibody in the combined use were extremely small, namely, 1/10 and 1/5 of the high dose of compound A (30 mg/kg) and the high dose of the anti-TNFα antibody (250 μg/mouse), respectively. However, the combined use of compound A and the anti-TNFα antibody exhibited a significantly strong anti-arthritic effect.

As is clear from the above results, a combined administration of the benzophenone derivative represented by the general formula [1] or the salt thereof and one or more TNFα inhibitors exhibits synergistic anti-arthritis effects, and thus it is useful for the treatment such as the cure and prevention of arthritis.

## INDUSTRIAL APPLICABILITY

A method of using a benzophenone derivative or a salt thereof and one or more TNFα inhibitors in combination is useful as a method for the treatment such as the cure or prevention of autoimmune diseases. A pharmaceutical composition containing these substances is useful for the treatment such as the cure or prevention of autoimmune diseases.

## Claims

1. A combination of a benzophenone derivative represented by the general formula below or a salt thereof and one or more TNFα inhibitors for use in a method of treatment of autoimmune diseases:

[Formula 1]

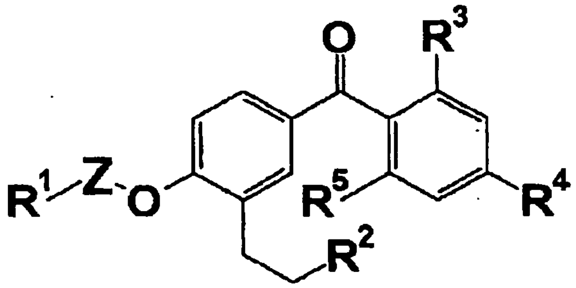

wherein

$R^1$ represents a heterocyclic group which may be substituted, a substituted phenyl group or an alkyl group which may be substituted;
Z represents an alkylene group which may be substituted;
$R^2$ represents a heterocyclic group which may be substituted, an alkoxycarbonyl or heterocyclic carbonyl group which may be substituted, or a carboxyl group which may be protected;
$R^3$ represents a hydrogen atom, a halogen atom, a cyano group, a nitro group, a carboxyl group which may be protected, a hydroxyl group which may be protected, an amino group which may be protected, a mercapto group, a carbamoyl group, or an alkyl, alkenyl, cycloalkyl, aryl, aralkyl, alkoxy, aryloxy, acyl, alkoxycarbonyl, aryloxycarbonyl, alkylthio, alkylsulfinyl, alkylsulfonyl, alkylamino, acylamino, alkylsulfonylamino, arylsulfonylamino or heterocyclic group which may be substituted;
$R^4$ represents a cycloalkyloxy group which may be substituted; and
$R^5$ represents a hydrogen atom, a halogen atom, or a hydroxyl group.

2. The combination for the use according to claim 1, wherein

$R^1$ represents a heterocyclic group which may be substituted, or a substituted phenyl group;
$R^2$ represents a carboxyl group which may be protected with an alkyl group;
$R^3$ represents a hydroxyl group which may be protected;
$R^5$ represents a hydrogen atom; and
Z represents an alkylene group.

3. The combination for the use according to claim 1 or 2, wherein $R^1$ represents a heterocyclic group which may be substituted; $R^2$ represents a carboxyl group; and $R^3$ represents a hydroxyl group.

4. The combination for the use according to claim 1, wherein the benzophenone derivative is a compound selected from 2-(4-morpholinyl)ethyl 3-(5-(4-(cyclopentyloxy)-2-hydroxybenzoyl)-2-((3-hydroxy-1,2-benzisoxazol-6-yl)methoxy)phenyl)propionate, 4-((2-(2-carboxyethyl)-4-(4-(cyclopentyloxy)-2-hydroxybenzoyl)phenoxy)methyl)benzoic acid, and 3-(5-(4-(cyclopentyloxy)-2-hydroxybenzoyl)-2-((4-(3-hydroxy-5-isoxazolyl)benzyl)oxy)phenyl)propionic acid.

5. The combination for the use according to claim 1, wherein the benzophenone derivative is 3-(5-(4-(cyclopentyloxy)-2-hydroxybenzoyl)-2-((3-hydroxy-1,2-benzisoxazol-6-yl)methoxy)phenyl)propionic acid.

6. The combination for the use according to claims 1 to 5, wherein the TNFα inhibitor is an anti-TNFα antibody.

7. The combination for the use according to claims 1 to 6, wherein the autoimmune diseases are arthritis diseases.

**8.** A pharmaceutical composition for the treatment of autoimmune diseases, which comprises a benzophenone derivative represented by the general formula below or a salt thereof and one or more TNFα inhibitors:

[Formula 2]

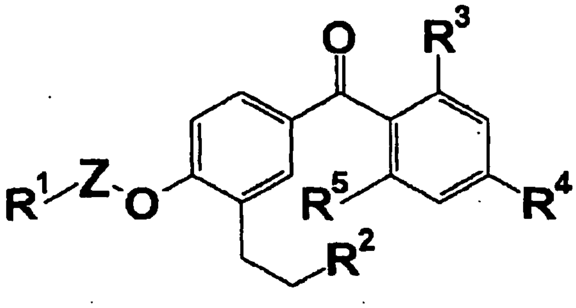

wherein

$R^1$ represents a heterocyclic group which may be substituted, a substituted phenyl group or an alkyl group which may be substituted;
Z represents an alkylene group which may be substituted;
$R^2$ represents a heterocyclic group which may be substituted, an alkoxycarbonyl or heterocyclic carbonyl group which may be substituted, or a carboxyl group which may be protected;
$R^3$ represents a hydrogen atom, a halogen atom, a cyano group, a nitro group, a carboxyl group which may be protected, a hydroxyl group which may be protected, an amino group which may be protected, a mercapto group, a carbamoyl group, or an alkyl, alkenyl, cycloalkyl, aryl, aralkyl, alkoxy, aryloxy, acyl, alkoxycarbonyl, aryloxycarbonyl, alkylthio, alkylsulfinyl, alkylsulfonyl, alkylamino, acylamino, alkylsulfonylamino, arylsulfonylamino or heterocyclic group which may be substituted;
$R^4$ represents a cycloalkyloxy group which may be substituted; and
$R^5$ represents a hydrogen atom, a halogen atom, or a hydroxyl group.

**9.** The pharmaceutical composition according to claim 8, wherein

$R^1$ represents a heterocyclic group which may be substituted, or a substituted phenyl group;
$R^2$ represents a carboxyl group which may be protected with an alkyl group;
$R^3$ represents a hydroxyl group which may be protected;
$R^5$ represents a hydrogen atom; and
Z represents an alkylene group.

**10.** The pharmaceutical composition according to claim 8 or 9, wherein

$R^1$ represents a heterocyclic group which may be substituted;
$R^2$ represents a carboxyl group; and
$R^3$ represents a hydroxyl group.

**11.** The pharmaceutical composition according to claim 8, wherein the benzophenone derivative is a compound selected from 2-(4-morpholinyl)ethyl 3-(5-(4-(cyclopentyloxy)-2-hydroxybenzoyl)-2-((3-hydroxy-1,2-benzisoxazol-6-yl)methoxy)phenyl)propionate, 4-((2-(2-carboxyethyl)-4-(4-(cyclopentyloxy)-2-hydroxybenzoyl)phenoxy)methyl)benzoic acid, and 3-(5-(4-(cyclopentyloxy)-2-hydroxybenzoyl)-2-((4-(3-hydroxy-5-isoxazolyl)benzyl)oxy)phenyl)propionic acid.

**12.** The pharmaceutical composition according to claim 8, wherein the benzophenone derivative is 3-(5-(4-(cyclopentyloxy)-2-hydroxybenzoyl)-2-((3-hydroxy-1,2-benzisoxazol-6-yl)methoxy)phenyl)propionic acid.

**13.** The pharmaceutical composition according to claims 8 to 12, wherein the TNFα inhibitor is an anti-TNFα antibody.

**14.** A kit comprising

(i) a benzophenone derivative as defined in any one of claims 1 to 5 or a salt thereof and
(ii) one or more TNFα inhibitors.

**15.** The kit according to claim 14, wherein the TNFα inhibitor is an anti-TNFα antibody.

**Patentansprüche**

**1.** Kombination aus einem Benzophenonderivat der allgemeinen Formel unten oder einem Salz desselben und einem oder mehreren TNFα-Inhibitoren zur Verwendung in einem Verfahren zur Behandlung von Autoimmunerkrankungen:

[Formel 1]

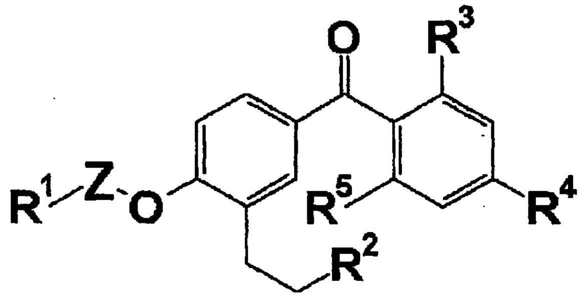

worin

$R^1$ für eine heterocyclische Gruppe, die substituiert sein kann, eine substituierte Phenylgruppe oder eine Alkylgruppe, die substituiert sein kann, steht;
Z für eine Alkylengruppe steht, die substituiert sein kann;
$R^2$ für eine heterocyclische Gruppe, die substituiert sein kann, eine Alkoxycarbonyl-oder Heterocyclus-Carbonylgruppe, die substituiert sein können, oder eine Carboxylgruppe, die geschützt sein kann, steht;
$R^3$ für ein Wasserstoffatom, ein Halogenatom, eine Cyanogruppe, eine Nitrogruppe, eine Carboxylgruppe, die geschützt sein kann, eine Hydroxylgruppe, die geschützt sein kann, eine Aminogruppe, die geschützt sein kann, eine Mercaptogruppe, eine Carbamoylgruppe oder eine Alkyl-, Alkenyl-, Cycloalkyl-, Aryl-, Aralkyl-, Alkoxy-, Aryloxy-, Acyl-, Alkoxycarbonyl-, Aryloxycarbonyl-, Alkylthio-, Alkylsulfinyl-, Alkylsulfonyl-, Alkylamino-, Acylamino-, Alkylsulfonylamino-, Arylsulfonylamino- oder heterocyclische Gruppe, die substituiert sein können, steht;
$R^4$ für eine Cycloalkyloxygruppe steht, die substituiert sein kann; und
$R^5$ für ein Wasserstoffatom, ein Halogenatom oder eine Hydroxylgruppe steht.

**2.** Die Kombination zur Verwendung gemäß Anspruch 1, worin

$R^1$ für eine heterocyclische Gruppe, die substituiert sein kann, oder eine substituierte Phenylgruppe steht;
$R^2$ für eine Carboxylgruppe steht, die mit einer Alkylgruppe geschützt sein kann;
$R^3$ für eine Hydroxylgruppe steht, die geschützt sein kann;
$R^5$ für ein Wasserstoffatom steht; und
Z für eine Alkylengruppe steht.

**3.** Die Kombination zur Verwendung gemäß Anspruch 1 oder 2, worin $R^1$ für eine heterocyclische Gruppe steht, die substituiert sein kann; $R^2$ für eine Carboxylgruppe steht; und $R^3$ für eine Hydroxylgruppe steht.

**4.** Die Kombination zur Verwendung gemäß Anspruch 1, worin das Benzophenonderivat eine Verbindung ist ausgewählt aus 2-(4-Morpholinyl)ethyl 3-(5-(4-(cyclopentyloxy)-2-hydroxybenzoyl)-2-((3-hydroxy-1,2-benzisoxazol-6-yl)methoxy)phenyl)propionat, 4-((2-(2-Carboxyethyl)-4-(4-(cyclopentyloxy)-2-hydroxybenzoyl)phenoxy)methyl)benzoesäure und 3-(5-(4-(Cyclopentyloxy)-2-hydroxybenzoyl)-2-((4-(3-hydroxy-5-isoxazolyl)benzyl)oxy)phenyl)propionsäure.

**5.** Die Kombination zur Verwendung gemäß Anspruch 1, worin das Benzophenonderivat 3-(5-(4-(Cyclopentyloxy)-2-hydroxybenzoyl)-2-((3-hydroxy-1,2-benzisoxazol-6-yl)methoxy)phenyl)propionsäure ist.

**6.** Die Kombination zur Verwendung gemäß einem der Ansprüche 1 bis 5, worin der TNF$\alpha$-Inhibitor ein anti-TNF$\alpha$-Antikörper ist.

**7.** Die Kombination zur Verwendung gemäß einem der Ansprüche 1 bis 6, worin die Autoimmunerkrankungen Arthritiserkrankungen sind.

**8.** Pharmazeutische Zusammensetzung zur Behandlung von Autoimmunerkrankungen, die ein Benzophenonderivat der allgemeinen Formel unten enthält oder ein Salz desselben und ein oder mehr TNF$\alpha$-Inbibitoren:

[Formel 2]

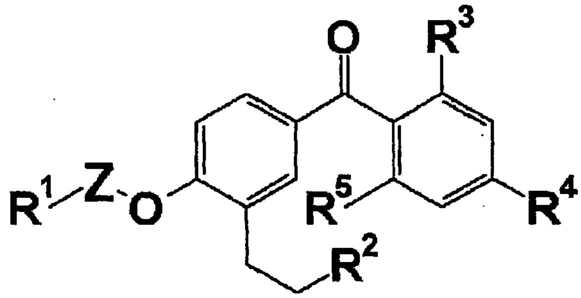

worin

$R^1$ für eine heterocyclische Gruppe, die substituiert sein kann, eine substituierte Phenylgruppe oder eine Alkylgruppe, die substituiert sein kann, steht;
Z für eine Alkylengruppe steht, die substituiert sein kann;
$R^2$ für eine heterocyclische Gruppe, die substituiert sein kann, eine Alkoxycarbonyl-oder Heterocyclus-Carbonylgruppe, die substituiert sein kann, oder eine Carboxylgruppe, die geschützt sein kann, steht;
$R^3$ für ein Wasserstoffatom, ein Halogenatom, eine Cyanogruppe, eine Nitrogruppe, eine Carboxylgruppe, die geschützt sein kann, eine Hydroxylgruppe, die geschützt sein kann, eine Aminogruppe, die geschützt sein kann, eine Mercaptogruppe, eine Carbamoylgruppe oder für eine Alkyl-, Alkenyl-, Cycloalkyl-, Aryl-, Aralkyl-, Alkoxy-, Aryloxy-, Acyl-, Alkoxycarbonyl-, Aryloxycarbonyl-, Alkylthio-, Alkylsulfinyl-, Alkylsulfonyl-, Alkylamino-, Acylamino-, Alkylsulfonylamino-, Arylsulfonylamino- oder heterocyclische Gruppe, die substituiert sein können, steht;
$R^4$ für eine Cycloalkyloxygruppe steht, die substituiert sein kann; und
$R^5$ für ein Wasserstoffatom, ein Halogenatom oder eine Hydroxylgruppe steht.

**9.** Die pharmazeutische Zusammensetzung gemäß Anspruch 8, worin

$R^1$ für eine heterocyclische Gruppe, die substituiert sein kann, oder eine substituierte Phenylgruppe steht;
$R^2$ für eine Carboxylgruppe steht, die mit einer Alkylgruppe geschützt sein kann;
$R^3$ für eine Hydroxylgruppe steht, die geschützt sein kann;
$R^5$ für ein Wasserstoffatom steht; und
Z für eine Alkylengruppe steht.

**10.** Die pharmazeutische Zusammensetzung gemäß Anspruch 8 oder 9, worin

$R^1$ für eine heterocyclische Gruppe steht, die substituiert sein kann;
$R^2$ für eine Carboxylgruppe steht; und
$R^3$ für eine Hydroxylgruppe steht.

**11.** Die pharmazeutische Zusammensetzung gemäß Anspruch 8, worin das Benzophenonderivat eine Verbindung ist ausgewählt aus 2-(4-Morpholinyl)ethyl 3-(5-(4-(cyclopentyloxy)-2-hydroxybenzoyl)-2-((3-hydroxy-1,2-benzisoxazol-6-yl)methoxy)phenyl)propionat, 4-((2-(2-Carboxyethyl)-4-(4-(cyclopentyloxy)-2-hydroxybenzoyl)phenoxy)methyl)benzoesäure und 3-(5-(4-(Cyclopentyloxy)-2-hydroxybenzoyl)-2-((4-(3-hydroxy-5-isoxazolyl)benzyl)oxy)phenyl)propionsäure.

**12.** Die pharmazeutische Zusammensetzung gemäß Anspruch 8, worin das Benzophenonderivat 3-(5-(4-(cyclopentyloxy)-2-hydroxybenzoyl)-2-((3-hydroxy-1,2-benzisoxazol-6-yl)methoxy)phenyl)propionsäure ist.

**13.** Die pharmazeutische Zusammensetzung gemäß einem der Ansprüche 8 bis 12, worin der TNFα-Inhibitor ein anti-TNFα-Antikörper ist.

**14.** Kit umfassend

(i) ein Benzophenonderivat wie in einem der Ansprüche 1 bis 5 definiert oder ein Salz davon und
(ii) ein oder mehrere TNFα-Inhibitoren.

**15.** Der Kit gemäß Anspruch 14, worin der TNFα-Inhibitor ein anti-TNFα-Antikörper ist.

## Revendications

**1.** Combinaison d'un dérivé de benzophénone représenté par la formule générale ci-dessous ou d'un sel de celui-ci et d'un ou plusieurs inhibiteur(s) de TNFα, pour utilisation dans une méthode de traitement de maladies auto-immunes :

[Formule 1]

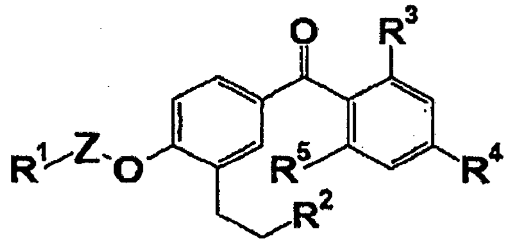

dans laquelle

$R^1$ représente un groupe hétérocyclique qui peut être substitué, un groupe phényle substitué, ou un groupe alkyle qui peut être substitué ;
Z représente un groupe alkylène qui peut être substitué ;
$R^2$ représente un groupe hétérocyclique qui peut être substitué, un groupe alcoxycarbonyle ou carbonyle hétérocyclique qui peut être substitué, ou un groupe carboxyle qui peut être protégé ;
$R^3$ représente un atome d'hydrogène, un atome d'halogène, un groupe cyano, un groupe nitro, un groupe carboxyle qui peut être protégé, un groupe hydroxyle qui peut être protégé, un groupe amino qui peut être protégé, un groupe mercapto, un groupe carbamoyle, ou un groupe alkyle, alcényle, cycloalkyle, aryle, aralkyle, alcoxy, aryloxy, acyle, alcoxycarbonyle, aryloxycarbonyle, alkylthio, alkylsulfinyle, alkylsulfonyle, alkylamino, acylamino, alkylsulfonylamino, arylsulfonylamino ou hétérocyclique qui peut être substitué ;
$R^4$ représente un groupe cycloalkyloxy qui peut être substitué ; et
$R^5$ représente un atome d'hydrogène, un atome d'halogène, ou un groupe hydroxyle.

**2.** Combinaison pour utilisation selon la revendication 1, dans laquelle

$R^1$ représente un groupe hétérocyclique qui peut être substitué, ou un groupe phényle substitué ;
$R^2$ représente un groupe carboxyle qui peut être protégé par un groupe alkyle ;
$R^3$ représente un groupe hydroxyle qui peut être protégé ;
$R^5$ représente un atome d'hydrogène ; et
Z représente un groupe alkylène.

**3.** Combinaison pour utilisation selon la revendication 1 ou 2, dans laquelle $R^1$ représente un groupe hétérocyclique qui peut être substitué ; $R^2$ représente un groupe carboxyle ; et $R^3$ représente un groupe hydroxyle.

**4.** Combinaison pour utilisation selon la revendication 1, dans laquelle le dérivé de benzophénone est un composé choisi parmi le 3-(5-(4-cyclopentyloxy)-2-hydroxybenzoyl)-2-((3-hydroxy-l,2-benzisoxazol-6-yl)-méthoxy)phényl)propionate de 2-(4-morpholinyl)éthyle, l'acide 4-((2-(2-carboxyéthyl)-4-(4-(cyclopentyloxy)-2-hydroxybenzoyl)phénoxy)méthyl)benzoïque, et l'acide 3-(5-(4-(cyclopentyloxy)-2-hydroxybenzoyl)-2-((4-(3-hydroxy-5-isoxazolyl)benzyl)oxy)phényl)propionique.

**5.** Combinaison pour utilisation selon la revendication 1, dans laquelle le dérivé de benzophénone est l'acide 3-(5-(4-(cyclopentyloxy)-2-hydroxybenzoyl)-2-((3-hydroxy-1,2-benzisoxazol-6-yl)méthoxy)phényl)-propionique.

**6.** Combinaison pour utilisation selon les revendications 1 à 5, dans laquelle l'inhibiteur de TNF$\alpha$ est un anticorps anti-TNF$\alpha$.

**7.** Combinaison pour utilisation selon les revendications 1 à 6, dan laquelle les maladies auto-immunes sont des maladies arthritiques.

**8.** Composition pharmaceutique pour le traitement de maladies auto-immunes, qui comprend un dérivé de benzophénone représenté par la formule générale ci-dessous ou un sel de celui-ci et un ou plusieurs inhibiteur(s) de TNF$\alpha$ :

[Formule 2]

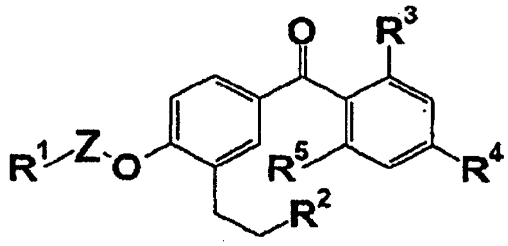

dans laquelle

$R^1$ représente un groupe hétérocyclique qui peut être substitué, un groupe phényle substitué, ou un groupe alkyle qui peut être substitué ;
Z représente un groupe alkylène qui peut être substitué ;
$R^2$ représente un groupe hétérocyclique qui peut être substitué, un groupe alcoxycarbonyle ou carbonyle hétérocyclique qui peut être substitué, ou un groupe carboxyle qui peut être protégé ;
$R^3$ représente un atome d'hydrogène, un atome d'halogène, un groupe cyano, un groupe nitro, un groupe carboxyle qui peut être protégé, un groupe hydroxyle qui peut être protégé, un groupe amino qui peut être protégé, un groupe mercapto, un groupe carbamoyle, ou un groupe alkyle, alcényle, cycloalkyle, aryle, aralkyle, alcoxy, aryloxy, acyle, alcoxycarbonyle, aryloxycarbonyle, alkylthio, alkylsulfinyle, alkylsulfonyle, alkylamino, acylamino, alkylsulfonylamino, arylsulfonylamino ou hétérocyclique qui peut être substitué ;
$R^4$ représente un groupe cycloalkyloxy qui peut être substitué ; et
$R^5$ représente un atome d'hydrogène, un atome d'halogène, ou un groupe hydroxyle.

**9.** Composition pharmaceutique selon la revendication 8, dans laquelle

$R^1$ représente un groupe hétérocyclique qui peut être substitué, ou un groupe phényle substitué ;
$R^2$ représente un groupe carboxyle qui peut être protégé par un groupe alkyle ;
$R^3$ représente un groupe hydroxyle qui peut être protégé ;
$R^5$ représente un atome d'hydrogène ; et
Z représente un groupe alkylène.

**10.** Composition pharmaceutique selon la revendication 8 ou 9, dans laquelle

$R^1$ représente un groupe hétérocyclique qui peut être substitué ;

$R^2$ représente un groupe carboxyle ; et
$R^3$ représente un groupe hydroxyle.

**11.** Composition pharmaceutique selon la revendication 8, dans laquelle le dérivé de benzophénone est un composé choisi parmi le 3-(5-(4-cyclopentyloxy)-2-hydroxybenzoyl)-2-((3-hydroxy-1,2-benzisoxazol-6-yl)-méthoxy)phényl)propionate de 2-(4-morpholinyl)éthyle, l'acide 4-((2-(2-carboxyéthyl)-4-(4-(cyclopentyloxy)-2-hydroxybenzoyl)phénoxy)méthyl)benzoïque, et l'acide 3-(5-(4-(cyclopentyloxy)-2-hydroxybenzoyl)-2-((4-(3-hydroxy-5-isoxazolyl)benzyl)oxy)phényl)propionique.

**12.** Composition pharmaceutique selon la revendication 8, dans laquelle le dérivé de benzophénone est l'acide 3-(5-(4-(cyclopentyloxy)-2-hydroxybenzoyl)-2-((3-hydroxy-1,2-benzisoxazol-6-yl)méthoxy)phényl)-propionique.

**13.** Composition pharmaceutique selon les revendications 8 à 12, dans laquelle l'inhibiteur de TNF$\alpha$ est un anticorps anti-TNF$\alpha$.

**14.** Kit comprenant

(i) un dérivé de benzophénone tel que défini dans l'une quelconque des revendications 1 à 5 ou un sel de celui-ci et
(ii) un ou plusieurs inhibiteur(s) de TNF$\alpha$.

**15.** Kit selon la revendication 14, dans lequel l'inhibiteur de TNF$\alpha$ est un anticorps anti-TNF$\alpha$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03042150 A


### Non-patent literature cited in the description


- The New England Journal of Medicine. *N. Engl. J. Med.,* 2006, vol. 355, 704-712
- The New England Journal of Medicine. *N. Engl. J. Med.,* 1996, vol. 334, 1287-1291
- **W. GREENE et al.** Protective Groups in Organic Synthesis. John Wiley & Sons, INC, 1999, 369-453
- **W. GREENE et al.** Protective Groups in Organic Synthesis. John Wiley & Sons, INC, 1999, 494-615
- **W. GREENE et al.** Protective Groups in Organic Synthesis. John Wiley & Sons, INC, 1999, 17-245